# EUROPEAN PATENT APPLICATION

(11) **EP 0 706 784 A1**
(43) Date of publication of application: **17.04.1996**
(21) Application number: 95202762.1
(22) Date of filing: 13.10.1995
(51) Int. Cl.: A61F 2/06

(54) **Stent which can be implanted into a body vessel**

(30) Priority: 13.10.1994 NL 9401690
(71) Applicant: CORDIS EUROPA N.V., NL-9301 LJ Roden (NL)
(72) Inventor: Glastra, Hendrik, NL-7535 PG Enschede (NL)
(74) Representative: 't Jong, Bastiaan Jacobus

(57) **Abstract**

The invention relates to a stent for implantation into a body vessel. The stent comprises a substantially tube formed frame element having an outer surface and defining a central lumen, at least one carrier element being connected to said frame element and containing a medically active material having a therapeutic effect on a human body.

## Description

The invention relates to a stent for implantation into a body vessel.

More specifically the invention relates to a stent for implantation into a body vessel which contains a medically active material having a therapeutic effect on a human body.

From the European patent application 0 433 011 an intra-arterial stent is known which incorporates or is coated with a radial isotope and is therefore radioactive and provides a source of radiation in situ to prevent restenonis following balloon angioplasty.

From the international patent application WO 94/15583 a medicament dispensing stent is known. The medicament is gradually released as the biodegradable polymer from which the stent is made, is absorbed by the body of the patient.

The invention has for its object to provide a stent of the kind set forth above with which a controlled release of a medically active material can be obtained.

This object is achieved with the stent as characterized in claim 1.

Due to the fact that a medically active material having a therapeutic effect on a human body is contained in at least one carrier element the gradual release of this medically active can be readily programmed by a proper embodiment of this carrier element. The release is not dependent on the embodiment of the stent as a hole as is the case with the prior art stent.

Preferred embodiments and further aspects of the invention are claimed in the subclaims.

The invention will be explained in greater detail with reference to the attached drawings.

Figure 1 shows a longitudinal cross-section of a stent carrying a substance emitting radiation.

Figure 2 shows a longitudinal cross-section of a stent which, after positioning of the stent, releases a substance which has a therapeutic effect and is taken up by the body fluids flowing past.

Figure 3 shows a longitudinal cross-section of another embodiment of a stent which has a similar action.

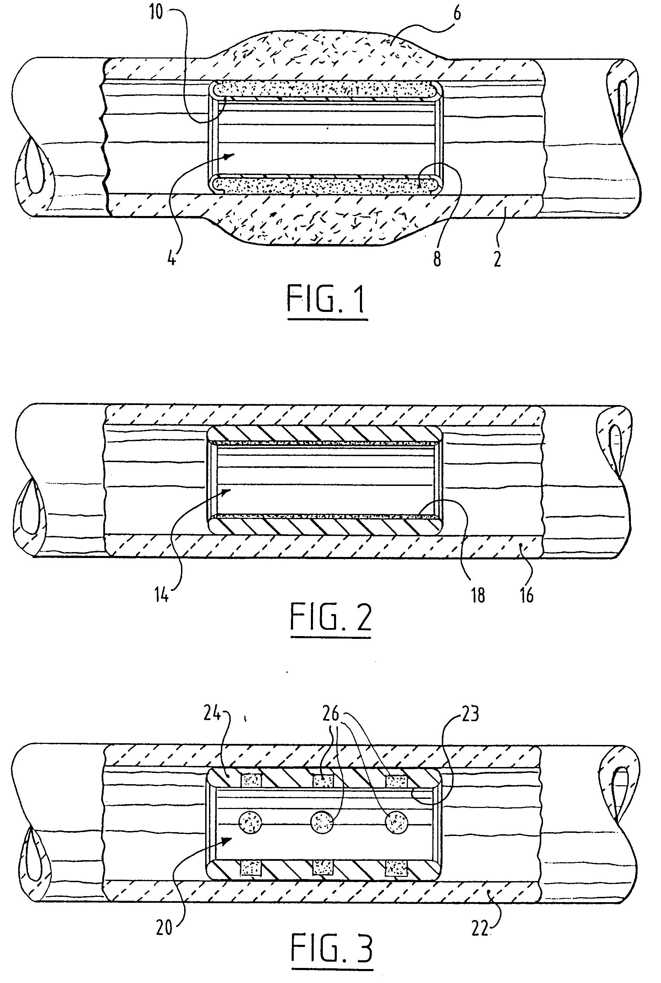

Figure 1 shows the body vessel 2 inside of which a stent 4 has been placed at the location of a tumour 6 surrounding the body vessel 2. This stent 4 is for instance of the type described in EP-A-0 521 572 and EP-A-0 617 930 respectively. The stent 4 comprises a substantially tube formed frame element 10 defining a lumen 5 and having an outer surface 7 being in contact with the body vessel 2. The frame element 10 defines a cavity 9 in which a carrier element 8 is connected containing a substance comprising a radioactive material. Quantity and action of this radioactive material can be determined accurately, so that also intensity and duration of the radiation are fixed. The continuous radiation acts on the tumour 6. The fact that the intensity and the direction of the radiation can be determined very accurately represents a marked improvement compared to the radiation techniques commonly employed.

If required, the frame element itself 10 can be impermeable to radiation.

Figure 2 shows an embodiment of a stent 14 arranged inside a body vessel 16, carrying, at the surface of the passage, a carrier element 18 comprising a substance which has a therapeutic effect, such as for instance a diluent. This substance is released gradually and evenly and taken up by the body fluids flowing through the body vessel 16 and past the stent, such as for instance blood.

Figure 3 shows an embodiment of a stent 20 received in a body vessel 22 of which the frame of the stent 24 has been provided with cavities 26 having a wall 27 which is permeable for the medically active material on the side of the inner surface 23. In these cavities a substance which has a therapeutic effect has been received which is released and taken up by the body fluids flowing through the body vessel 22 and past the stent 20.

It may be clear that in addition to the examples described above, a great many other embodiments and applications are possible.

## Claims

1. Stent for implantation into a body vessel, comprising a substantially tube formed frame element having an outer surface and defining a central lumen, at least one carrier element being connected to said frame element and containing a medically active material having a therapeutic effect on a human body.

2. Stent according to claim 1, wherein said frame element comprises at least one cavity containing the medically active material.

3. Stent according to claim 2, said cavity having a wall which is at least partly permeable for the medically active material.

4. Stent according to claim 3, wherein the at least partly permeable wall of the cavity is a wall bounding the lumen.

5. Stent according to claim 3, wherein the at least partly permeable wall of the cavity is a wall forming the outer surface.

6. Stent according to claim 1, wherein said medically active material is a radiation source of ionizing radiation.

7. Stent according to claim 1, wherein said medically active material is a diluent.
